# EUROPEAN PATENT SPECIFICATION

(11) **EP 3 919 009 B1**
(45) Date of publication and mention of the grant of the patent: **28.06.2023**
(21) Application number: 20823366.8
(22) Date of filing: 10.04.2020
(51) Int. Cl.: A44C 7/00, A44C 15/00

(54) **PIERCING NOSE STUD AND PIERCING APPARATUS**
PIERCING-NASENSTECKER UND PIERCING-VORRICHTUNG
PIERCING NASAL ET APPAREIL DE PERÇAGE

(30) Priority: 13.06.2019 CN 201910511001
(43) Date of publication of application: 08.12.2021
(73) Proprietor: Cheng, Bo, NanChang, Jiangxi 330029 (CN)
(72) Inventor: Cheng, Bo, NanChang, Jiangxi 330029 (CN)
(74) Representative: Huang, Liwei
(86) International application number: PCT/CN2020/084095
(87) International publication number: WO 2020/248685

(56) References cited:
- CN-U- 208 610 027
- DE-U1- 9 419 830
- GB-A- 2 310 378
- US-A1- 2005 268 652

## Description

### Technical Field of the Invention

The present application belongs to the technical field of ornaments, and particularly relates to a perforating apparatus.

### Background of the Invention

It is a long-standing tradition of human beings to wear decorative nose studs perforating nose wings. A simpler method of perforating the nose wing is to pierce the nose wing with a hollow needle, and then wear the nose stud thereon by running through a hole formed by the hollow needle. This method takes a long time to operate, causes severe pain, and also has high technical requirements for operators.

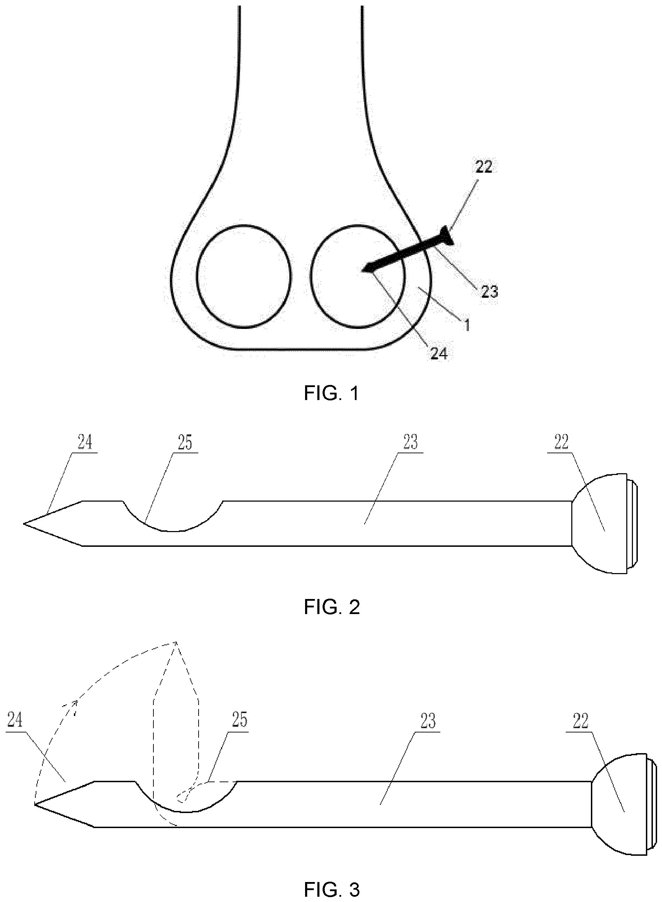

At present, the commonly used nose stud, which is a stud with a needle tip, is disposable product. The needle tip 24 pierces the nose wing 1 directly. As shown in FIG. 1, the needle tip 24 of the nose stud is larger than a needle bar 23 in terms of diameter, so that the needle tip 24 is stuck in the nostril after the nose stud runs through the nose wing 1 to avoid the nose stud falling off. This nose stud has two deficiencies: first, the needle tip 24 that stays in the nostril often pricks tissues in the nasal cavity and causes pain due to inevitable movements of the nose stud; second, as the needle tip 24 is larger than the needle bar 23 in terms of diameter, the needle bar 23 cannot completely squeeze the wound caused by the needle tip 24 during perforating, resulting in wound bleeding after the perforating operation.

CN208610027U provides a perforating apparatus according to the preamble of claim 1, comprising a cavity body, a piston body, a nose stud chuck, and a nose stud for perforating, wherein the piston body is of an L-shaped structure with one end sliding into the cavity body, the nose stud chuck is movably clamped on the cavity body and the nose stud chuck clamps the nose stud for perforating.

### Summary of the Invention

The present application provides a perforating apparatus as defined in the claim 1.

Optionally, the bendable portion comprises a recess with the axial direction thereof not parallel to that of the needle bar.

Optionally, the axial direction of the recess is perpendicular to that of the needle bar.

Optionally, a top end of the needle tip is located outside an axis of the needle bar.

Optionally, the top end of the needle tip and an open side of the recess are both located on the same side of the axis; or, the needle tip and the needle bar are of a hollow structure.

Optionally, the needle tip forms an eccentric structure relative to the needle bar, and an axis of the needle tip is parallel to that of the needle bar.

Optionally, the recess is an annular recess coaxial with the needle bar.

Optionally, the needle tip and the needle bar are of equal outer diameter.

The nose stud used for perforating provided by the present application comprises a needle tip and a needle bar, wherein the needle tip is connected to one end of the needle bar, a bendable structure is provided at a joint which bends when the needle tip is subjected to a resistance force not in the axial direction of the needle bar under a force applied by the needle bar. As a bendable structure is provided at the joint between the needle tip and the needle bar, the needle tip can bend relative to the needle bar after perforating the nose to avoid damaging tissues in the nasal cavity.

### Brief Description of the Drawings

FIG. 1 is a schematic diagram of a nose stud used for perforating nose wings in the prior art;
FIG. 2 is a structural diagram of the nose stud used for perforating according to an embodiment of the present application;
FIG. 3 is a schematic diagram of the nose stud used for perforating deformed by force according to the embodiment of the present application;
FIG. 4 is a structural diagram of a nose stud used for perforating according to another embodiment of the present application;
FIG. 5 is a perspective view of the nose stud used for perforating in FIG. 4 according to the embodiment of the present application;
FIG. 6 is a structural diagram of a nose stud used for perforating according to a third embodiment of the present application;
FIG. 7 is a structural diagram of a nose stud used for perforating according to a fourth embodiment of the present application;
FIG. 8 is a structural diagram of a nose stud used for perforating according to a fifth embodiment of the present application;
FIG. 9 is a structural diagram of a perforating apparatus according to an embodiment of the present application;
FIG. 10 is a sectional view of the perforating apparatus in FIG. 9 according to the embodiment of the present application;
FIG. 11 shows a state of the perforating apparatus in use according to the embodiment of the present application; and
FIG. 12 shows a state of the perforating apparatus after perforating the nose wing according to the embodiment of the present application,
   in which:
   1: nose wing; 22: nose stud head; 23: needle bar; 24: needle tip; 25: notch; 201: cavity body; 202: piston body; 203: spring; 204: nose stud chuck; 206: upper cavity; 207: guide rail; 208: top; 209: front wall; 210: rear wall; and 211: circular recess.

### Detailed Description of the Invention

Referring to FIGS. 1 to 8, a nose stud used for perforating is provided according to an embodiment of the present application, comprising a needle tip 24 and a needle bar 23, wherein the needle tip 24 is connected to one end of the needle bar 23, and an easilybendable portion, which bends when the needle tip 24 is subjected to a resistance force under a force applied by the needle bar 23, is provided at a joint.

In use, the needle bar 23 applies force to the needle tip 24, and the needle tip 24 pierces the nose wing 1 until the needle bar 23 is located in a hole formed by the needle tip 24 piercing the nose wing 1. When the needle tip 24 is subjected to a resistance force, the resistance force will cause thebendable portion at the joint between the needle tip 24 and the needle bar 23 to bend. In this way, the needle tip 24 bent to one side is less likely to prick tissues in the nasal cavity, thus alleviating pain. At the same time, the bent needle tip 24 forms a blocking structure to prevent the nose stud from falling off the nose wing 1.

The above bendable portion refers to a portion that is more likely to bend without deformation caused by fracture than the needle bar and the needle tip under the action of a stress transmitted hereto by a force in the direction from the needle bar to the needle tip.

For example, the easily bendable portion comprises a recess, the axial direction of the recess is non-parallel to the axial direction of the needle bar. When the needle tip 24 is subjected to a non-axial resistance, the needle tip 24 is more likely to bend at the recess, especially when the axial direction of the recess is perpendicular to that of the needle bar 23.

Optionally, a top end of the needle tip 24 is located outside the axis of the needle bar 23 and on an open side of the recess. In this way, when a top end of the needle tip 24 is subjected to a counter-acting force, the needle tip 24 is more likely to buckle at the recess.

### Embodiment 1

As shown in FIGS. 2 and 3, the nose stud used for perforating is of an integrated structure consisting of a needle tip 24, a needle bar 23 and a nose stud head 22, and a notch 25 is provided at a joint between the needle tip 24 and the needle bar 23. After the needle tip 24 pierces the nose wing 1, the needle tip 24 is subjected to a resistance force that deviates slightly, and the needle tip 24 bends to an open side of the notch 25 at the notch 25 until it bends to 90 degrees, as shown in FIG. 3.

### Embodiment 2

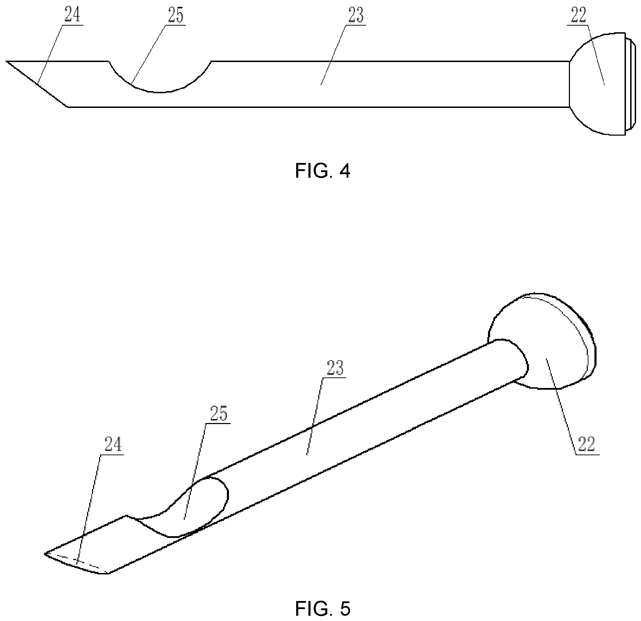

Unlike Embodiment 1, the needle tip 24 is of an inclined structure. As shown in FIGS. 4 and 5, the top end of the needle tip 24 and anopen side of the notch 25 are located on the same side. The force applied by the needle bar 23 to the needle tip 24 will cause the needle tip 24 to bend at the notch 25.

### Embodiment 3

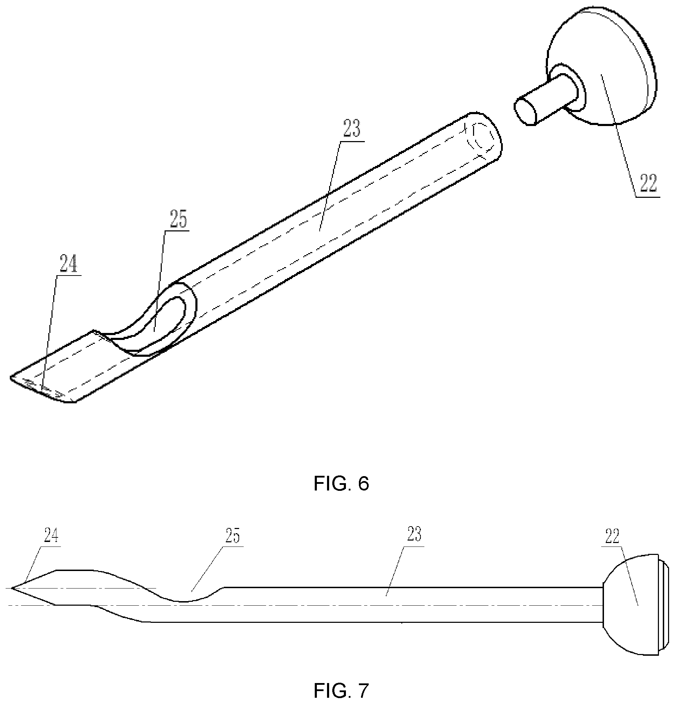

Unlike Embodiment 2, the needle tip 24 and the needle bar 23 are of an integrated hollow structure, and the nose stud head 22 is a moving piece. As shown in FIG. 6, when the needle bar 23 applies force to the needle tip 24, the needle tip 24 bends at the notch 25 under the action of counter-acting force.

### Embodiment 4

Unlike Embodiment 1, the needle tip 24, with an axis parallel to that of the needle bar 23, is of an eccentric structure relative to the needle bar 23. As shown in FIG. 7, the needle tip 24 is deflected to one side. When the needle bar 23 applies force to the needle tip 24, the needle tip 24 bends at the notch 25 under the action of counter-acting force.

### Embodiment 5

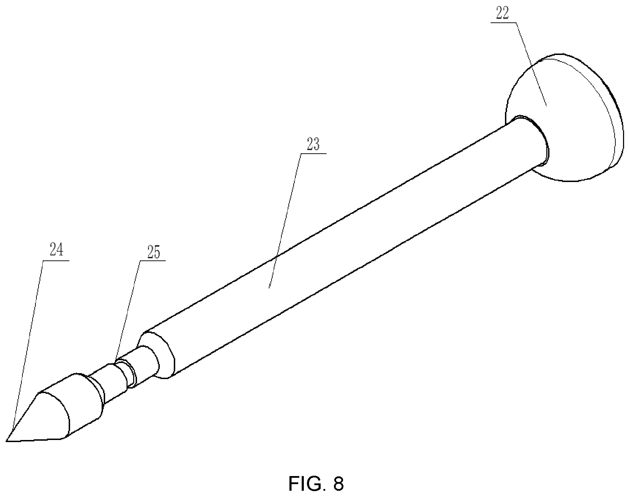

Unlike Embodiment 1, the recess is an annular recess coaxial with the needle bar. As shown in FIG. 8, when the needle bar 23 applies force on the needle tip 24, the needle tip 24 bends at the annular recessunder the action of counter-acting force applied by the inclined plane as the counter-acting force is not in the direction of the axis of the needle bar 23.

In production practice, the above structure can be implemented in a variety of ways. The needle bar 23 and the needle tip 24 may be integrated; the needle bar 23 and the nose stud head 22 may be connected by glue or threads and may also be integrated.

In production practice, the needle tip 24 and the needle bar 23 may be of equal outer diameter, and a hole formed by the needle tip 24 on the nose wing 1 will be blocked by the needle bar 23 to avoid wound bleeding.

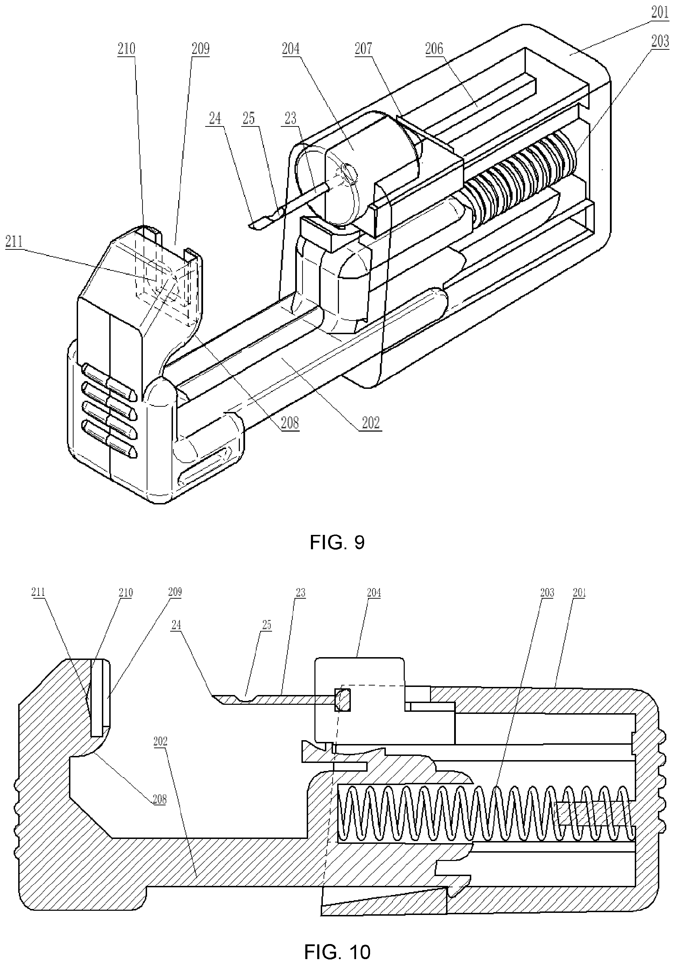

Referring to FIGS. 9 to 12, a perforating apparatus is provided according to an embodiment of the present application, comprising the nose stud used for perforating described above.

Specifically, the perforating apparatus comprises a cavity body 201, a piston body 202 and a nose stud chuck 204. The piston body 202 is of an L-shaped structure with one end sliding into the cavity body 201 and the other end having an inclined plane; the nose stud chuck 204 clamping the nose stud used for perforating and directly facing the inclined plane is movably clamped on the cavity body 201.When the piston body 202 slides into the cavity body 201, the needle tip 24 of the nose stud used for perforating bends upon contact with the inclined plane.

The cavity body 201 is of a structure consisting of an upper cavity 206 and a lower cavity. A guide rail 207, on which the nose stud chuck 204 is clamped and can move, is provided in the upper cavity 206. One end of the piston body 202 slides in the lower cavity, and a spring 203 is provided between the piston body 202 and the cavity body 201. At the other end, a top 208, which is a recess structure with one side open, directly faces the nose stud chuck 204, the open side is a front wall 209, and a conical recess or bulge 211 is provided at the center of a rear wall 210 to form an inclined surface structure in contact with the needle tip 24.

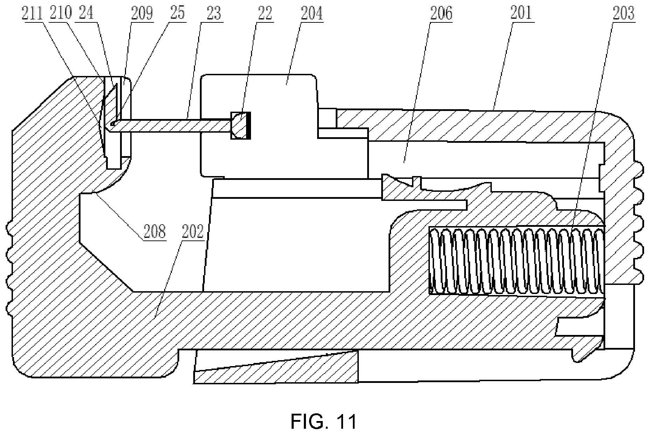

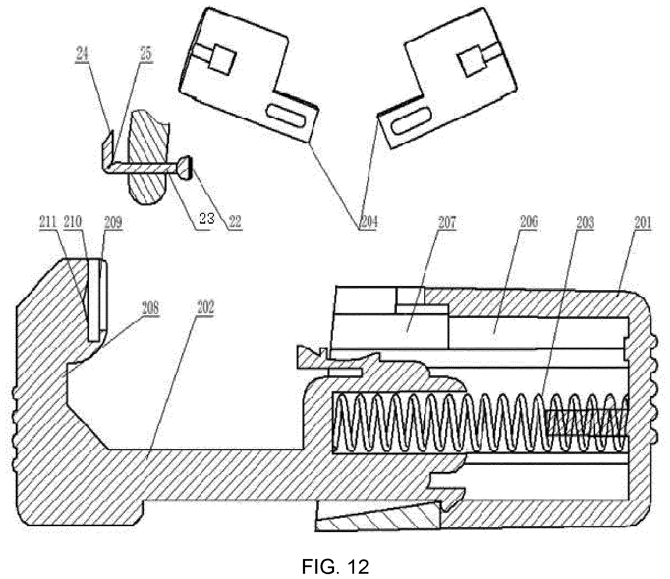

FIGS. 11 and 12 show an operating principle of the perforating apparatusadopting the nose stud used for perforating. When the cavity body 201 and the piston body 202 are pinched by hand and approached, the spring 203 is compressed, and the needle tip piercesthe nose wing 1 and passes through the opening ofthe front wall 209 into the conical recess or onto bulge 211 of the rear wall 210. The needle tip 24 bends at a notch 25 under the action of a thrust force to form a bending structure with an included angle of about 90 degrees with the needle bar 23. When the pinching force applied by hand is removed, the cavity body 201 and the piston body 202 are separated under the action of the spring 203, the nose stud chuck 204 is disengaged from the guide rail 207 and falls off the nose stud head 22, and finally the nose stud remains on the nose wing 1. At this moment, the perforating process of the nose stud is completed.

It should be readily understood by those of skill in the art that the above technical features may be freely combined within the scope of the claims.

## Claims

1. A perforating apparatus, comprising a cavity body (201), a piston body (202), a nose stud chuck (204), and a nose stud for perforating,
wherein the piston body (202) is of an L-shaped structure with one end sliding into the cavity body (201), the nose stud chuck (204) is movably clamped on the cavity body (201) and the nose stud chuck (204) clamps the nose stud for perforating, the nose stud for perforating comprises a needle tip (24) and a needle bar (23),
wherein
the needle tip (24) is connected to one end of the needle bar (23), wherein the connection portion comprises a bendable portion, which bends when the needle bar applies a force to the needle tip and the needle tip (24) is subjected to a resistance force;
**characterized in that**
the piston body (202) of the L-shaped structure has the other end equipped with an inclined plane, the nose stud chuck (204) directly faces the inclined plane; and when the piston body (202) slides into the cavity body (201), the needle tip (24) of the nose stud for perforating bends upon contact with the inclined plane.

2. The perforating apparatus according to claim 1, wherein the bendable portion of the nose stud comprises a recess wherein an axial direction of the recess is not parallel to an axial direction of the needle bar (23).

3. The perforating apparatus according to claim 2, wherein the axial direction of the recess is perpendicular to the axial direction of the needle bar (23).

4. The perforating apparatus according to claim 1, 2 or 3, wherein a top end of the needle tip (24) is located outside an axis of the needle bar (23).

5. The perforating apparatus according to claim 4, wherein the top end of the needle tip (24) and an open side of the recess are both located on the same side of the axis; or, the needle tip (24) and the needle bar (23) are of a hollow structure.

6. The perforating apparatus according to claim 4, wherein the needle tip (24) forms an eccentric structure relative to the needle bar (23), and an axis of the needle tip (24) is parallel to the axis of the needle bar (23).

7. The perforating apparatus according to claim 1, wherein the bendable portion of the nose stud comprises a recess, wherein the recess is an annular recess coaxial with the needle bar (23).

8. The perforating apparatus according to claim 1, wherein the needle tip (24) and the needle bar (23) are of equal outer diameter.

## Patentansprüche

1. Perforationsvorrichtung, umfassend einen Hohlraumkörper (201), einen Kolbenkörper (202), ein Nasenbolzenfutter (204) und einen Nasenbolzen zum Perforieren,
wobei der Kolbenkörper (202) von einer L-förmigen Struktur ist, wobei ein Ende in den Hohlraumkörper (201) gleitet, das Nasenbolzenfutter (204) bewegbar an den Hohlraumkörper (201) geklemmt ist und das Nasenbolzenfutter (204) den Nasenbolzen zum Perforieren klemmt, der Nasenbolzen zum Perforieren eine Nadelspitze (24) und eine Nadelstange (23) umfasst, wobei
die Nadelspitze (24) mit einem Ende der Nadelstange (23) verbunden ist, wobei der Verbindungsabschnitt einen biegbaren Abschnitt umfasst, der sich biegt, wenn die Nadelstange eine Kraft auf die Nadelspitze ausübt und die Nadelspitze (24) einer Widerstandskraft ausgesetzt wird;
**dadurch gekennzeichnet, dass**
das andere Ende des Kolbenkörpers (202) der L-förmigen Struktur mit einer geneigten Ebene ausgestattet ist, das Nasenbolzenfutter (204) der geneigten Ebene direkt zugewandt ist; und wenn der Kolbenkörper (202) in den Hohlraumkörper (201) gleitet, sich die Nadelspitze (24) des Nasenbolzens zum Perforieren bei Kontakt mit der geneigten Ebene biegt.

2. Perforationsvorrichtung nach Anspruch 1, wobei der biegbare Abschnitt des Nasenbolzens eine Aussparung umfasst, wobei eine axiale Richtung der Aussparung nicht parallel zu einer axialen Richtung der Nadelstange (23) ist.

3. Perforationsvorrichtung nach Anspruch 2, wobei die axiale Richtung der Aussparung senkrecht zu der axialen Richtung der Nadelstange (23) ist.

4. Perforationsvorrichtung nach Anspruch 1, 2 oder 3, wobei sich ein oberes Ende der Nadelspitze (24) außerhalb einer Achse der Nadelstange (23) befindet.

5. Perforationsvorrichtung nach Anspruch 4, wobei sich das obere Ende der Nadelspitze (24) und eine offene Seite der Aussparung beide auf derselben Seite der Achse befinden; oder die Nadelspitze (24) und die Nadelstange (23) von einer hohlen Struktur sind.

6. Perforationsvorrichtung nach Anspruch 4, wobei die Nadelspitze (24) eine exzentrische Struktur relativ zu der Nadelstange (23) bildet und eine Achse der Nadelspitze (24) parallel zu der Achse der Nadelstange (23) ist.

7. Perforationsvorrichtung nach Anspruch 1, wobei der biegbare Abschnitt des Nasenbolzens eine Aussparung umfasst, wobei die Aussparung eine ringförmige Aussparung ist, die koaxial mit der Nadelstange (23) ist.

8. Perforationsvorrichtung nach Anspruch 1, wobei die Nadelspitze (24) und die Nadelstange (23) von gleichem Außendurchmesser sind.

## Revendications

1. Appareil de perforation, comprenant un corps de cavité (201), un corps de piston (202), un mandrin à piercing nasal (204) et un piercing nasal destiné à perforer,
ledit corps de piston (202) étant d'une structure en forme de L avec une extrémité coulissant dans le corps de cavité (201), ledit mandrin à piercing nasal (204) étant serré de manière mobile sur le corps de cavité (201) et ledit mandrin à piercing nasal (204) serrant le piercing nasal destiné à perforer, ledit piercing nasal destiné à perforer comprenant une pointe d'aiguille (24) et une barre d'aiguille (23),
ladite pointe d'aiguille (24) étant reliée à une extrémité de la barre d'aiguille (23), ladite partie de raccordement comprenant une partie pliable, qui se plie lorsque la barre d'aiguille applique une force sur la pointe d'aiguille et que la pointe d'aiguille (24) est soumise à une force de résistance ;
**caractérisé en ce que**
l'autre extrémité du corps de piston (202) de la structure en forme de L est équipée d'un plan incliné, le mandrin à piercing nasal (204) faisant directement face au plan incliné ; et lorsque le corps de piston (202) coulisse dans le corps de cavité (201), la pointe d'aiguille (24) du piercing nasal se plie lors du contact avec le plan incliné.

2. Appareil de perforation selon la revendication 1, ladite partie pliable du piercing nasal comprenant un évidement, la direction axiale de l'évidement n'étant pas parallèle à la direction axiale de la barre d'aiguille (23).

3. Appareil de perforation selon la revendication 2, ladite direction axiale de l'évidement étant perpendiculaire à la direction axiale de la barre d'aiguille (23).

4. Appareil de perforation selon la revendication 1, 2 ou 3, l'extrémité supérieure de la pointe d'aiguille (24) étant située en dehors de l'axe de la barre d'aiguille (23).

5. Appareil de perforation selon la revendication 4, ladite extrémité supérieure de la pointe d'aiguille (24) et un côté ouvert de l'évidement étant tous deux situés du même côté de l'axe ; ou, ladite pointe d'aiguille (24) et ladite barre d'aiguille (23) étant d'une structure creuse.

6. Appareil de perforation selon la revendication 4, ladite pointe d'aiguille (24) formant une structure excentrique par rapport à la barre d'aiguille (23), et l'axe de la pointe d'aiguille (24) étant parallèle à l'axe de la barre d'aiguille (23).

7. Appareil de perforation selon la revendication 1, ladite partie pliable du piercing nasal comprenant un évidement, ledit évidement étant un évidement annulaire coaxial avec la barre d'aiguille (23).

8. Appareil de perforation selon la revendication 1, ladite pointe d'aiguille (24) et ladite barre d'aiguille (23) étant d'un diamètre externe égal.
